# EUROPEAN PATENT APPLICATION

(11) **EP 0 972 828 A1**
(43) Date of publication of application: **19.01.2000**
(21) Application number: 98401554.5
(22) Date of filing: 24.06.1998
(51) Int. Cl.: C12N 5/06, C12N 7/02

(54) **Process for the in vitro replication of HCV**

(71) Applicant: INSTITUT NATIONAL DE LA SANTE ET DE LA RECHERCHE MEDICALE (INSERM), 75654 Paris Cédex 13 (FR)
(72) Inventor: Rumin, Sylvie, 35000 Rennes (FR); Inchauspe, Genevievè, 69003 Lyon (FR); Trepo, Christian, 69500 Bron (FR); Gripon, Philippe, 35000 Rennes (FR)
(74) Representative: Grosset-Fournier, Chantal Catherine

(57) **Abstract**

The invention relates to a use of a culture medium containing
- one or several mammalian plasma or sera,
- a chemical or biological compound having an antioxydative property and/or differentiating property, such as DMSO, retinoic acid, vitamin, for example vitamin E, or selenium,
- and/or one or several corticoids
   for the *in vitro* hepatitis C virus replication in primary mammalian hepatocytes.

## Description

The invention relates to a process for the *in vitro* replication of HCV.

Hepatitis C, that is a non A non B hepatitis, is a transmissible disease that is induced by Hepatitis C Virus (HCV), the major cause of acquired hepatitis by transfusion. Hepatitis C is distinguishable from other forms of viral-associated liver diseases, such as hepatitis A, hepatitis B or hepatitis D. Evidence of HCV infection is found throughout the world. Infections are usually chronic.

HCV was the first hepatotropic virus to be isolated by the mean of molecular biology techniques (1). Viral genomic sequences were cloned before the viral particle itself could be visualized (14).

HCV is a single-stranded positive-sense RNA virus of about 9.5 kb in length, translated in a unique polyprotein precursor of approximatively 3,000 amino acids. Because of its genomic organization and its presumed replication mode, HCV was classified in a new genus within the *Flaviviridae* family (32), which includes two other genera : the flaviviruses and the animal pestiviruses and classical swine fever virus. All members of this family have enveloped virions that contain a positive-strand RNA encoding all known virus-specific proteins via translation of a single open reading frame.

Many techniques have been developed to diagnose an infection by HCV. For example, immunodiagnostic assays have been developed for detecting antibodies directed against HCV proteins in the serum of patients. Synthesis of cDNA by reverse transcription of the viral RNA and amplification by PCR have been also used for detecting HCV genome, as an indirect measure of potentially infectious virus in sera from chronically infected humans or experimentally infected chimpanzees. On the basis of cloning an HCV gene, hybridization techniques with a DNA probe have also been used.

It is submitted that existing diagnostic methods to some extent lack of sensitivity and/or specificity and/or praticability. For example, with the probe hybridization method, it is impossible to distinguish a virus with less infectivity versus a virus with high infectivity. Consequently, it is necessary albeit impracticle to inoculate a chimpanzee with the virus to be tested and test the resultant infection thereof in the chimpanzee.

The demand for sensitive and specific methods for identifying and screening carriers of HCV is important for public health. A solution for addressing these problems could be to perform a highly efficient *in vitro* culture system of HCV allowing persistent *in vitro* virus propagation, specifically to study the mechanisms of replication of the virus, to test neutralizing antibodies as well as antiviral drugs and to develop biological materials and tests for diagnostic. Indeed, an *in vitro* culture system would provide a source of material, such as viral polypeptides or nucleotide sequences, which could be used for the development of diagnostic assays and/or vaccines. In addition, they could be used in drug screening and for developing antiviral agents. However, a reliable and robust *in vitro* culture method has until now not been available.

*In vivo,* evidence of HCV replication in cells from the immune system has been documented (15, 26, 27, 38) but remains matter of controversy (23, 25, 31). HCV has also been shown to be able to replicate in vitro in lymphoid cell lines and peripheral blood mononuclear cells, but at very low levels and/or for short period of time (3, 16, 33, 34, 40, 41).

*In vitro* propagation of HCV in liver cells has been as least as difficult to implement in hepatic cell lines as in primary hepatocyte cultures (10, 13, 17, 22, 39). In chimpanzee hepatocytes for instance, Landford et al. could not demonstrate the production of viral particles in the supernatant of infected cells, suggesting that a complete HCV replication cycle was not achieved (22). Although, Ito et al. confirmed the maintenance of HCV replication in human hepatocyte cultures established from *in vivo* chronic HCV carriers and suggested a passage of infection, technical problems relating to the propagation of the virus still persist (13). More recently, Iacovacci et al. also demonstrated *in vitro* infection of human fetal hepatocytes (17). Overall, major differences between lymphoid and hepatic cells were (i) low levels of replication and selection of a particular quasispecies in lymphoid cell lines (34) (ii) higher replication level in hepatocytes (17, 22) and maintenance of all quasispecies present *in vivo* (13). In these models, evidence of HCV replication always required the use of nested-PCR, and production of infectious viral particles in the cell supernatant was rarely demonstrated.

To date, *in vitro* infection assays using normal adult human hepatocytes in primary cultures have not been described.

The objective of the present invention is to provide an efficient *in vitro* infection system using hepatocytes and particularly mammalian and more particularly human hepatocytes, which avoid the problems cited hereabove.

Culture conditions are defined for allowing the maintenance of HCV replication for at least three months post-infection. One embodiment involves the use of PCR amplification techniques, for example PCR, in three different regions of the viral genome (5' and 3' non coding regions, envelope glycoprotein E2). Sufficient viral production was obtained to analyze and quantify HCV RNA by single-round PCR and standardized validated commercial assays but also to measure core antigen in the supernatants of infected cells. The reproducibility of the method on hepatocytes from different liver donors using extensively characterized inocula is also taught. Finally, quasispecies selected after in vitro infection were analyzed in the supernatants of infected cells and compared in cultures established from three different liver donors.

The techniques described herein are validated by experimental protocols.

Liver resection samples were obtained from three patients undergoing partial hepatectomy for metastatic liver tumor. They were negative for HIV, HBV and HCV infections by serological assays (ELISA HIV1 HIV2.3 from Abbott and HIV1-2-3 Vironostika assay, E1A assays from Abbott for the detection of HBsAg, anti-HBc and anti-HBs antibodies, and anti-HCV antibodies detection with HCV 3.0 kit from Abbott). Experimental procedures were performed in accordance to the French Laws and Regulation, with the informed patient's consent.

The invention relates to the use of a culture medium containing :
- one or more mammalian plasma or sera,
- a chemical or biological compound having an antioxidative property and/or differentiating property, such as DMSO (dimethyl sulfoxide), retinoic acid, vitamin, for example vitamin E, or selenium,
- and/or one or more corticoids
   for the *in vitro* hepatitis C virus replication in primary mammalian hepatocytes.

The term "compound having antioxidative property" corresponds to substances which are opposed to oxidation of lipids, proteins, and nucleic acids in a cell or a cell line submitted to oxidative stress conditions. These substances can
- block the productions of free radicals, and/or
- trap the free radicals, and/or
- inactivate free radicals.

The term "compound having differentiating property" corresponds to a substance which induces and/or maintains specific functions of differentiated cells (i.e. production of albumin, cytochrome p450 and acute phase protein in hepatocytes).

The invention relates to a process for the *in vitro* hepatitis C virus replication in primary mammalian hepatocytes comprising the step of incubation of a primary mammalian hepatocyte culture medium containing
- one or more mammalian plasma or sera,
- a chemical or biological compound having an antioxidative property and/or differentiating property, such as DMSO, retinoic acid, vitamin, for example vitamin E, or selenium,
- and/or one or more corticoids

In the process of the invention, DMSO is advantageously used as an ingredient in the viral hepatocyte culture medium.

According to an advantageous embodiment, the process of the invention is characterized in that the culture medium contains :
- one or several mammalian plasma or sera,
- a chemical or biological compound having an antioxydative property and/or differentiating property, such as DMSO, retinoic acid, vitamin, for example vitamin E, or selenium.

According to an another advantageous embodiment, the process of the invention is characterized in that the culture medium is a primary human hepatocyte culture medium containing a non-infected human plasma or serum (i.e. a human serum not infected by HCV), DMSO, and a corticoid chosen from among hydrocortisone derivatives such as hydrocortisone hemisuccinate, or dexamethasone.

According to an advantageous embodiment, the process of the invention is characterized in that:
- the ratio of non-infected human plasma or serum is from about 1 % to about 10 % by volume of said culture medium, in particular about 1 %, to about 5%, and preferably about 2%,
- the ratio of DMSO in said culture medium is from about 0.5 % to about to about 3 % by volume, in particular about 2 %,
- the concentration of the corticoid is from about 10⁻⁷M to about 10⁻⁴M, in particular about 10⁻⁶M.

According to an advantageous embodiment, the process of the invention is characterized in that the culture medium also contains :
- antibiotics such as penicillin, streptomycin, and/or
- interferon, and/or
- mammalian insulin, and/or
- foetal calf serum, and/or
- a basic medium for *in vitro* cell cultures, such as William's E medium, or a culture medium adapted for long term culture of epithelial cells.

According to an advantageous embodiment, the process of the invention is characterized in that the culture medium does not contain PEG.

According to an another advantageous embodiment, the process of the invention is characterized in that :
- the HCV titer of the inoculum is from about 10⁻³ to about 100 genomic equivalents per hepatocyte of the culture, in particular from 0.1 to about 10 and in particular from about 1 to about 10 genomic equivalents/hepatocyte,
- and the resulting HCV titer in the culture medium after incubation with said inoculum, is from about 5.10⁻⁵ to about 1 genomic equivalent/hepatocyte/day.

According to an advantageous embodiment in the process of the invention, the hepatocytes are initially seeded in the culture medium at the ratio of about 1.5 x10⁵ to about 2.10⁵ cells/cm².

The invention also relates to the use of the culture system of the invention for the *in vitro* screening of anti-HCV drugs.

The term "anti-HCV drugs" designates natural or chemical or synthetic substances having the property of partially or totally inhibiting the viral propagation in the organism. It can include substances inhibiting the infection processus (blocking of the receptor such as antibodies - and/or internalization process) and/or inhibiting replication (such as antisens) and/or secretion of viral particles.

The invention also relates to the use of the culture system of the invention, for the *in vitro* diagnosis of HCV, for example for the development of antibodies directed to HCV and/or synthetic polypeptides or recombinant proteins and/or nucleotide probes.

The invention also relates to the use of a process of the invention, for the preparation of vaccines against HCV, for example the testing of neutralizing antibodies, cross-neutralization.

The invention also relates to HCV infected mammalian hepatocyte culture medium containing
- one or more mammalian plasma or sera,
- a chemical or biological compound having an antioxydative property and/or differentiating property, such as DMSO, retinoic acid, vitamin, for example vitamin E, or selenium,
- and/or one or more corticoids.

The invention also relates to HCV infected mammalian hepatocyte culture medium containing
- one or more mammalian plasma or sera,
- a chemical or biological compound having an antioxydative property and/or differentiating property, such as DMSO, retinoic acid, vitamin, for example vitamin E, or selenium.

The invention also relates to HCV infected mammalian hepatocyte culture medium as above defined, characterized in that said culture medium is a human hepatocyte culture medium containing a non-infected human serum or plasma, DMSO, and a corticoid chosen among hydrocortisone derivatives such as hydrocortisone hemisuccinate, or dexamethasone.

The invention also relates to HCV infected mammalian hepatocyte culture medium as above defined, characterized in that:
- the ratio of non-infected human plasma or serum is from about 1 % to about 10 % by volume of said culture medium, in particular about 1 %, to about 5 %, and preferably about 2 %,
- the ratio of DMSO in said culture medium is from about 0.5 % to about to about 3 % by volume , in particular about 2 %,
- the concentration of the corticoid is from about 10⁻⁷M to about 10⁻⁴M, in particular about 10⁻⁶M.

The invention also relates to HCV infected mammalian hepatocyte culture medium as above defined, wherein HCV infected hepatocytes survive for at least 4 months.

The invention also relates to HCV particles obtained according to the process above defined.

### FIGURE LEGENDS:

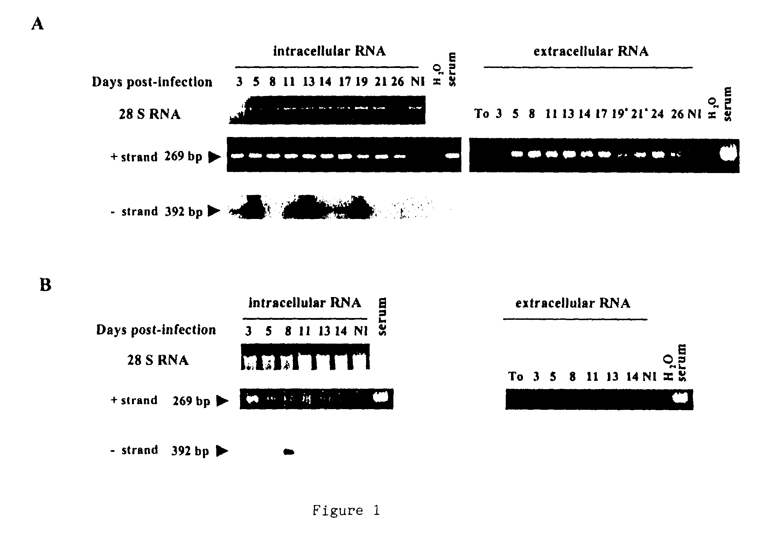

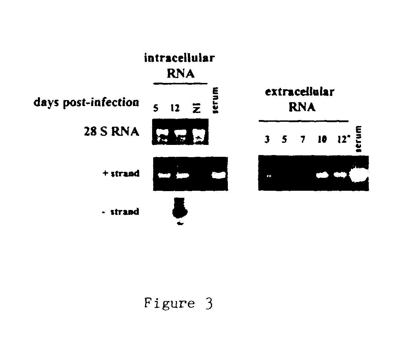

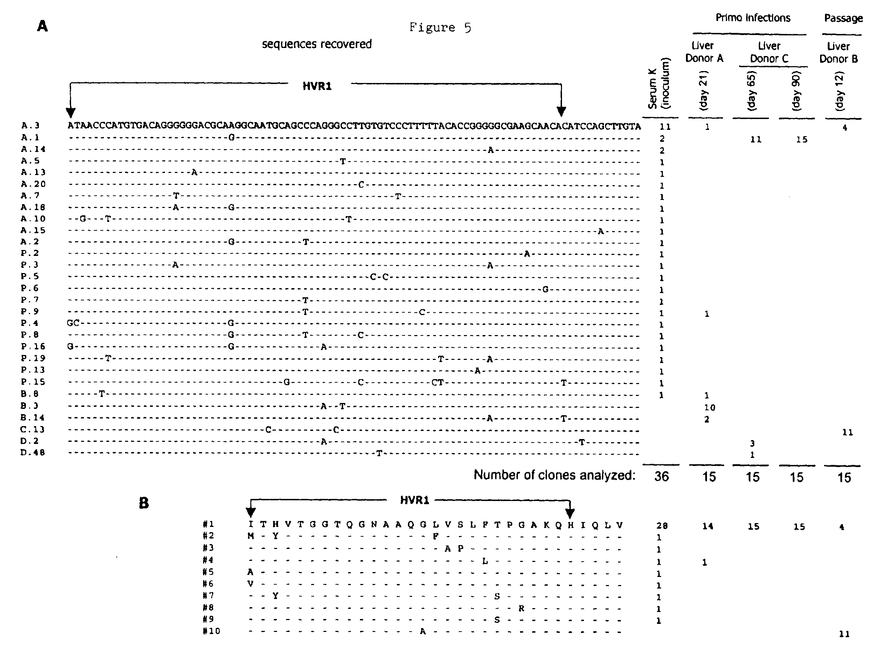

Figure 1: Kinetics of detection of positive and negative-strand HCV RNA in cells and supernatants of primary hepatocytes cultured in two different media of the present invention. Infections were performed using the serum K03 (genotype 1a) on cultures established from liver donor A. Cultures were maintained either in the presence of 2% human serum, 2% DMSO and 3.5 10-7 M hydrocortisone (A) or in the presence of 10% FCS and 3.5 10-5 M hydrocortisone (B). Ethidium bromide staining of 28 S RNA is shown to demonstrate the integrity of total cellular RNA after extraction procedure. Ethidium bromide staining of single round PCR products obtained in the 5'NCR (269 bp) are shown for the positive-strand HCV RNA. Agarose gel electrophoresis followed by Southern blot hybridization was conducted for the negative-strand according to the "Tagged PCR" method described by Landford (product size 392 bp, 22). * indicates the supernatants used for the passage of infection shown in Fig. 3 and for quasispecies analysis documented in Fig. 5. Negative controls included PCR amplification performed from culture medium before infection (To), non infected cells (NI) and water (H2O). RNA isolated from HCV positive serum was used as positive control for positive-strand RNA detection and as negative control for negative-strand RNA detection.

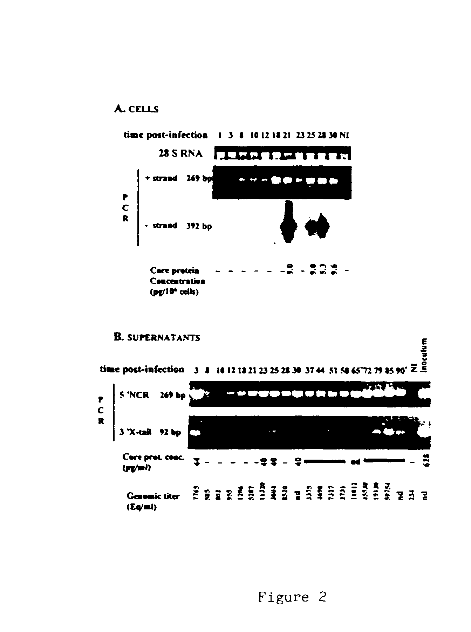

Figure 2 : Kinetics of HCV replication in long-term cultures of human hepatocytes maintained in the presence of 2% human serum, 2% DMSO and 3.5 10-7 M hydrocortisone. Infections were performed on cultures established from liver donor C. Cellular extracts (A) and culture supernatants (B) were analyzed. 28 S RNA and positive-strand as well as negative-strand RNA amplification products were obtained as in the Fig. 1. 3' X-tail amplification (92 bp product) was performed as described by Umlauft et al. (48). Viral titers obtained by Monitor test (Roche DiagnosticSystem) and core protein concentration (45) are shown. Negative controls were RNA isolated from mock-infected cells and supernatants (NI). (-) means that core protein concentration was below the threshold of detection, and nd means that these titers were not determined. * indicates the supernatants used for quasispecies analysis.
Figure 3 : Kinetics of positive and negative-strand viral RNA in the cells and supernatants of hepatocytes infected with supernatants of cultures infected with serum K03: passage of the infection. Cells were from liver donor B. 28 S RNA and positive-strand as well as negative-strand RNA amplification products were obtained as in the Fig. 1. * shows the supernatant used for quasispecies analysis.

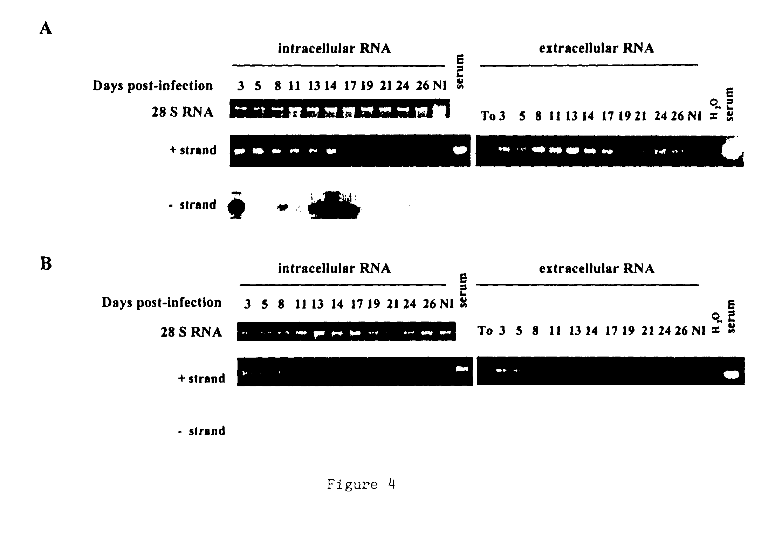

Figure 4 : Kinetics of detection of positive and negative-strand HCV RNA in cells and supernatants of primary hepatocyte cultures infected with serum S05 (genotype 1b) (A) and L02 (genotype 2a/2c) (B) in the culture medium containing 2% human serum, 2% DMSO and 3.5 10-7 M hydrocortisone. Cultures were established from liver donor A. Viral positive and negative-strand RNA were analyzed by PCR as described in Fig. 1. Controls included PCR amplification performed from culture medium before infection (To), non infected cells (NI), water (H2O) and serum from HCV chronic carrier.
Figur 5 : HVR1 nucleotide sequences of HCV and numbers of clones recovered from the serum K03 (inoculum) and supernatants of primary cultures of human hepatocytes from three different liver donors at various times post-infection. Cloning and sequencing were performed as described herebelow. Nucleotide sequences from nucleotides 1490 to 1583 according to strain H (11) are shown in (A). The corresponding amino acid sequences are shown (B). Horizontal bars indicate nucleotides or amino acids identical to those of the consensus sequence.

### Example 1 : Primary hepatocyte cultures

Cells were isolated by a two-step collagenase (Boehringer Mannheim) perfusion procedure as previously described (9). A 30% percoll (Sigma) gradient was realized on isolated cells in order to remove dead cells. Freshly isolated hepatocytes were seeded in 6-well dishes at a density of 1.5 to 2x10⁵ viable cells/cm2. This high density is preferred to obtain long-term survival of primary hepatocytes (37). Adhesion was performed overnight in William's E medium (with Glutamax I, Sigma) complemented with 100 UI/ml penicillin and streptomycin (Gibco), 5 g/l bovine insulin (Sigma) and 10% heat inactivated fetal calf serum (FCS) (USDA batches, Eurobio). After seeding, medium was replaced by William's E supplemented with antibiotics and insulin as described above, plus either 2% dimethyl sulfoxide (Sigma), 2% normal human serum and 3.5 x 10-7 M hydrocortisone hemisuccinate (Roussel, France), or 10% FCS and 3.5 x 10-5 M hydrocortisone hemisuccinate. Normal human serum was obtained from bleeds of non-infected patients suffering from hemochromatosis with their informed consent.

### Example 2 : In vitro infection with HCV

Three days after seeding, cultures were incubated overnight at 37°C with 50 µl of the indicated serum diluted in 1.5 ml of medium devoid of normal human serum. Mock-infected cells were incubated with 50 µl of human non-infectious serum. The inoculum was removed and cell monolayers were washed three times with PBS and refed with the medium described above. Medium was changed every 2-3 days until harvest. It is to be noted that the inoculum which can be used in the present invention is a HCV infected serum having substantially similar characteristics with those of serum K03, as defined in Table 2 herafter.

### Example 3 : Extraction of nucleic acids and cDNA synthesis

At the time of harvest, the medium was collected and several aliquots stored at - 70°C. Cultures were washed in phosphate-buffered saline (PBS), trypsinized with 0.5 g/l trypsin - 0.2 g/l EDTA (Gibco) in order to remove adsorbed virions, and pelleted at 4°C. Paired cellular pellets were stored at -80°C until used. Intracellular total RNA as well as viral RNA contained in serum and supernatants were isolated using High Pure RNA Isolation Kit™ , according to the manufacturer's instruction (Boehringer Mannheim). A DNAse digestion of the sample is included in this procedure. Approximately 0.75 x 106 hepatocytes (half a dish) or 500 µl of supernatant (one third) or 150 µl of serum were extracted and collected in a final volume of 50 µl of water. Intracellular RNA integrity and homogeneity were checked by submitting 5 µl of total cellular RNA to electrophoresis on 1 % agarose gel containing ethidium bromide.

During all the extraction steps and RT-PCR amplification, guidelines of Kwok and Higuchi (20) for the prevention of PCR contamination were carefully followed. Standard procedure were performed as follows : ten microliters of RNA (corresponding to approximately 1µg of total RNA or 100 µl of supernatant) were preheated at 70°C with 0.1 µM of primer and reverse transcription was performed during 1 h at 42°C in a reaction mixture containing RT-buffer 1X (Gibco BRL), 10 mM dithiothreitol, 1 mM dNTP, 20 U RNAsin (Promega), and 200 U MMLV RT (Gibco BRL). For 3'X-tail detection, cDNA synthesis was performed at 52°C for 50 mm in a reaction mixture containing obtained cDNA was submitted to PCR amplification, after denaturation at 97°C during either 10 mm or 30 mm for detection of positive and negative-strand RNA respectively. Briefly, reaction mixture consisted of 1X Taq buffer, 1.5 mM MgC12, 0.1 mM dNTP, 1 µM of each primer (Table 1) and 1.25 U Taq DNA polymerase (Perkin Elmer). Two different techniques were used for the amplification of the positive-strand RNA. PCR amplification of the 5'NCR was adapted from Li et al. (28), using single-round amplification with primers NC3 and NC4. NC3 and NC4 possess 32 and 81 percents homology respectively with Bovine Viral Diarrhea Virus, another member of the Flaviviridae family that was found to be frequently contamining FCS batches (49). Absence of detection of Bovine Viral Diarrhea Virus was confirmed in the samples. PCR conditions consisted of an initial step of 95°C for 5 min, 40 cycles of 95°C for 1 min, 64°C for 1 min, 72°C for 2 min, and a final extension step of 72°C for 10 min. In the second amplification of the 3' X-Tail was performed according to a single-step PCR method described by Umlauft et al. (48). Specificity of amplification products obtained in both techniques of detection of the positive-strand RNA was demonstrated using Southern blot hybridization with oligonucleotidic internal probes. Specific detection of the negative-strand RNA was performed using a method described by Landford et al. (22). The use of a Tag during PCR amplification was also shown to avoid artefactual amplification of the incorrect strand in our hands. Two nucleotides were added in 5' end of the primer initially described by Landford et al. for better amplification and specificity (see Table 1). After denaturation of reverse transcriptase and destruction of residual RNA in the cDNA preparation, PCR amplification was performed as following : 95°C for 5 min, 35 cycles of 94°C for 1 min 20 sec, 64°C for 2 min, 72°C for 3 min, and a final extension step at 70°C for 10 min. Typically, one-fourth of the PCR product was analyzed by agarose gel. Southern blot hybridization with a DIG-labeled probe internal to the PCR primers was performed in the Tag method according to the manufacturer's instructions (DIG-High prime DNA labeling and detection starter kit II,™ Boehringer).

Primers for the amplification of the Hypervariable Region 1 (HVR1) of the HCV genome were synthesized on the basis of the published 1a genotype sequence (11). Single-round PCR and DNA polymerases with high fidelity were used to avoid artefactual mutation during quasispecies analysis (44). Primers HVR1 and HVR2 were used for PCR amplification (see table 1). Two different enzymes with 3' exonuclease activities were compared for their efficacy of amplification and for their fidelity. Thirty five cycles were performed at 94°C, 30 sec, 65°C, 30 sec , 68°C, 1 min 30 sec with ELONGase™ (Gibco) or at 94°C, 1 min, 65°C, 1 min, 72°C, 2 min with Pfu (Stratagene), both after initial denaturation of the sample at 95°C. Sequences obtained from the serum after single round PCR amplification with either Pfu or ELONGase DNA polymerases, gave similar results (data not shown). Therefore, experiments described thereafter were performed using the ELONGase , because this mix of enzymes has a better efficacy than Pfu polymerase.

Alternate amplification techniques could of course be utilized.

### Example 4 : Cloning of HVR1 region

RT-PCR products from serum K03 or supernatants of cultures infected with this serum were purified with Wizard PCR-Prep (TM) (Promega) and subcloned into cloning vector PCR-Script TM Amp SK (+) (Stratagene) by the recommended procedure. A minimum of fifteen independent colonies were picked up, and the cDNA clones were sequenced in both directions with an Applied Biosystems 373A sequencer and Taq Dye Terminators according to Applied Biosystems protocol. The sequencing comparison were performed with Sequence Navigator software (Applied Biosystems).

### Example 5 : Virological assays

Quantification of the amount of HCV RNA was performed by Amplicor HCV Monitor™ (Roche Diagnostic System), based on a simplified RNA extraction and a single, combined reverse transcription and amplification reaction, mediated by thermus thermophilus DNA polymerase. Supernatants were processed directly. HCV RNA titers were also determined in some supernatants and in sera by the QuantiplexTM HCV RNA 2.0 Assay (bDNA, Chiron). Genotypes were determined by the Inno-Lipa HCV II test (TM) (Innogenetics, Belgium), GBV-C co-infection by the LCx test (TM) (Roche), anti-HCV antibodies (anti-C, E, NS3, NS4, NS5) by the Inno-Lia HCV ABIII test (Innogenetics). Anti-E1 and E2 antibody titers were kindly determined by E. Depla (Innogenetics). Results shown in Table 2 are indicated as following : (-): negative at serum dilution 1/20; (+) : positive at serum dilution 1/20; and (+ + +): positive at serum dilution 1/200. Neutralization of Binding (NOB) titers were determined by Rosa and Abrignani (35). This assay is based on the neutralization of binding of purified envelope glycoprotein 2 expressed in mammalian cells to human cells. According to this assay, high NOB titers are > 1/2000 i.e. a 1/2000 dilution of the serum has neutralizing activity against binding of purified E2 on target cells.

### Example 6 : Quantitative analysis of HCV core protein concentration

The concentration of HCV core protein was measured by the sandwich FEIA in cellular extracts and supernatants of infected cells according to a procedure described previously (45, 46), with some modifications in the steps of processing samples. Briefly, the step of precipitating proteins in samples by polyethylene glycol was omitted because the protein was expressed as pg/ml using standard of c11 recombinant HCV core protein. The limit of detection was calculated as 40 pg/ml for supernatants and 5.3 pg/106 cells for cell extracts.

### GenBank/EMBL accession numbers

Nucleotide sequences from the HVR1 region obtained in this study have been submitted to DDBJ/EMBL/GenBank DNA data bases with accession numbers AF045896 to AF045924.

### Example 7 : Maintenance of HCV replication

*In vitro* HCV infected hepatocytes were analyzed up to 3 months post-infection (Fig. 2). In this experiment, positive-strand RNA was barely detectable before day 8 and day 12 post-infection in the cells (Fig. 2A) and in the supernatants (Fig. 2B) respectively. Negative-strand RNA detection occurred also later in this experiment than in previous ones (day 21 post-infection, Fig. 2A). Kinetics of RNA replication seemed therefore to vary from one donor to the other. Quantitative analysis of the secreted viral RNA was carried out using Amplicor HCV Monitor test (Fig. 2B). After an initial release of particles from the inoculum (still detected at day 3), genomic titers in the supernatants of infected cells were very low from day 8 to 10 post-infection, and then gradually increased during the 3 months of culture, ranging from 955 at day 12 post-infection to about 60,000 genomic equivalents/ml at day 90. This correlated well with the intensity of PCR products in ethidium bromide stained electrophoresis gels. Remarkably, an enhancement of approximately one Log occurred after 2 months of culture. One interpretation of this observation could be that HCV replication adapted to in vitro culture. Quantitative analysis was also performed using bDNA assay. Titers obtained confirmed those obtained using the Monitor test, although their range was one Log higher, similarly to what is usually observed for sera.

In order to further assess the presence of HCV RNA in the supernatants of long-term cultures of infected hepatocytes, the positive-strand HCV RNA was amplified using primers located in the extreme 3' NCR of HCV genome : the highly conserved tail of 98 nucleotides in length extending HCV genome after the poly(U) stretch referred to as 3' X-tail (19, 47). PCR products obtained using this approach resulted in a weaker and less systematic detection, likely due to a lower sensitivity of this single-round PCR technique (Fig. 2B). However, these data demonstrated the presence of the full length HCV genome released in culture medium following in vitro infection. Since this amplification product was hardly detectable before 23 days in culture, and reached its highest level by the end of the culture, this further indicated that HCV RNA detected in the supernatants of infected cells did not represent carryover of infectious particles from the very high titer inoculum K03. Moreover, genomic titers obtained after 3 months of culture were approximately 10-fold higher than those observed at day 3.

The production of capsid antigen in infected cells was successfully demonstrated by a quantitative method previously described (45) (Fig. 2A and B). Capsid antigens could be detected both in the cellular extracts (concentration range from 5.1 to 9.2 pg/106 cells) and supernatants of infected cells (40 pg/ml). These data further demonstrated that HCV capsid protein synthesis did occur in this in vitro infection model.

### Passage of the infection to naive hepatocytes

Detection of both viral RNA and capsid antigen in the supernatant of infected cells suggested the release of viral particles. To demonstrate that it was indeed the case, their infectivity was tested on naive cultures. Supernatants from cultures infected with serum K03 (Liver donor A, Fig. 1) were pooled from day 19 to 21 post-infection and incubated with cells obtained from another donor (liver donor B). Intra and extracellular RNA were extracted at various times post-infection. Results are shown in Fig. 3.

Positive-strand RNA was detected in the infected cells at days 5 and 12 post-infection, while the negative-strand RNA could only be detected at day 12 post-infection. In this case, release of viral particles from the inoculum was barely detected. De novo produced viral RNA appeared around 10 days after infection. As a control, direct infection of cells from the same liver donor was performed with the inoculum K03 ; it induced earlier replication. These results confirm that infectious viral particles were secreted in the supernatants of *in vitro* infected human hepatocytes.

### Influence of sera characteristics and liver cell donors on in vitro infectivity

In order to assess the reproducibility of the experiments, three different sera (K03, S05, and L02, genotypes 1a, 1b and 2a/c respectively) were tested for their infectivity on hepatocyte cultures established from the livers of three donors (A, B and C). Results for liver donor A are illustrated in Fig. 1A (for serum K03) and Fig. 4A and B (for sera S05 and L02 respectively), while overall results are described in Table 2. Kinetics of replication obtained with serum S05 were similar to that obtained with serum K03 (Fig. 1A and 4A). In cells infected with serum S05 however, level of positive-strand RNA seemed to decrease after day 17 post-infection, both intra and extra-cellularly, while it remained high at least up to 26 days post-infection in cells infected with serum K03. It should also be noticed that with this serum (genomic titer 17.8 MEq/ml), no release of viral RNA from the inoculum was observed at day 3 post-infection. In cells infected with serum L02, replication could not be maintained after 8 days post-infection, as positive-strand RNA was barely detectable at later times and negative-strand RNA was never detected (Fig. 4B). Overall, serum K03 was infectious for cells obtained from all three livers, serum S05 was infectious in 2/3 experiments, while replication could never be established from serum L02 (Table 2). According to these results, infectivity seemed directly correlated with genotype 1 and high genomic titer, and possibly inversely correlated with the anti-envelope antibody titers (no antibodies detected at serum dilution 1/20 for serum K03, antibodies detected at serum dilution 1/20 for serum S05, and detected up to dilution 1/200 for serum L02). Infectivity of serum S05 also appeared to depend on the liver donor.

Determination of the quasispecies present in serum K03 and in infected cultures established from three different liver donors.

Quasispecies is defined as a population of closely related heterogeneous sequences (quasipecies) centenred around one dominant sequence (referred to as the master sequence) in a single infected individual.

Quasispecies present in the inoculum K03 and in the supernatants of in vitro infected cultures established from three different livers were analyzed by cloning and sequencing the HCV hypervariable region 1 (HVR1). Quasispecies distribution was analyzed at both the nucleotide (Fig. 5A) and the amino acid level (Fig. 5B). Twenty four different sequences (from 36 clones analyzed) were found in serum K03 (Fig. 5A). One nucleotide sequence (A.3) was dominant in this serum, since 11/36 clones were identical (30%), while the remaining ones were minor (representing 2.7 to 5.5 % of the total clones obtained) differing from each other in 1 to 5 nucleotides. Interestingly, this major quasispecies detected in the inoculum (A.3) was only poorly represented in supernatants of infected cells and cultures derived from all three livers preferentially supported replication of minor variants found in the inoculum (sequences B.3 for liver donor A, A. 1 for donor C and C. 13 for donor B). In all three cases, such variants represented less than 5.5% of circulating virions from serum K03. Major quasispecies recovered from the cultures from donors A and B were not found in the inoculum, indicating that it represented less than 2.7 % of the circulating virions. When supernatant collected from donor A (21 days post-infection) was used to infect naive cells from donor B (passage), the major quasispecies recovered after passage of the infection (sequence C. 13) was different from the one found in hepatocytes from donor A (sequence B.3), therefore representing less than 6.6% of clones analyzed at day 21 in donor A. This could be one explanation for the observed delay in the establishment of viral replication after passage (Fig. 3). Overall, these results showed that different quasispecies were selected in cultures established from different livers, and that in all three cases reported here the quasispecies selected in the culture were minor components of the inoculum.

Since an increase in viral RNA secretion was observed after two months in culture (infection of hepatocyte culture from donor C, illustrated in Fig. 2), an analysis was made whether this was due to the adaptation of certain quasispecies to in vitro replication. To answer this question, the HVR1 sequence of viral RNA released in the supernatant of this long-term culture at days 65 and 90 post-infection, i.e. before and after the increase of viral titer was analyzed. This analysis showed that a least 3 distinct quasispecies were maintained after 2 months of culture (sequences A. 1, D.2 and D.48). In contrast, only one HVR1 sequence was detected at the end of this culture (A. 1). This could suggest that a selection of a quasispecies adapted to this donor occurred before day 65 and amplification of this minor subgroup went on thereafter.

The demonstration of an efficient replication over 3 months in the present culture medium using single-round RT-PCR, and quantification of the produced viral genomic RNA were achieved using a commercially available HCV RNA quantification kit (Monitor, Roche). Another marker of productive viral infection is the de novo synthesis of viral antigens. The concentration of core protein in the serum of chronically infected patients has been shown to be a reliable marker of *in vivo* HCV replication (46). The release of core protein in the supernatant of infected cells is a demonstration of efficient *in vitro* propagation of the virus. Extensive characterization was made of the sera used for in vitro infection. None of the sera characteristics analyzed (genotype, genomic titer, anti-envelope E1 and E2 antibody titer and NOB titer) were reliable predictive factors of serum infectivity, when considered separately. Interestingly however, reproducibility of infection was obtained on cultures from three liver donors with a genotype 1, very high genomic titer serum containing no detectable anti-envelope proteins and no neutralizing antibodies in the NOB assay.

In the described *in vitro* infection model of the present invention, a selection of distinct viral quasispecies from the same initial inoculum used to infect cultures established from different liver donors, was observed.

The present invention provides an efficient culture system for HCV replication and long-term propagation which can be achieved using *in vitro* infected adult primary human hepatocytes. In the model, genomic titer in the supernatant reaches 60,000 genomic equivalents/ml.

The described system will be useful for the analysis of the first steps of interaction of the viral particle with cells, the development of neutralization assays, the possible identification of HCV cellular receptor and the development of diagnostics assays. It is also particularly well suited for studies on antivirals, because (i) high level replication allows easy detection of viral genomic sequences by several single-step PCR methods or commercial quantitative kits, (ii) sufficient duration of replication is maintained for evaluation of potential resistance, (iii) hepatocytes are the site for metabolism of xenobiotics, and therefore of antiviral compounds and (iv) the use of normal hepatocytes from human origin will also allow a relevant estimation of cytotoxicity and pharmacological properties.

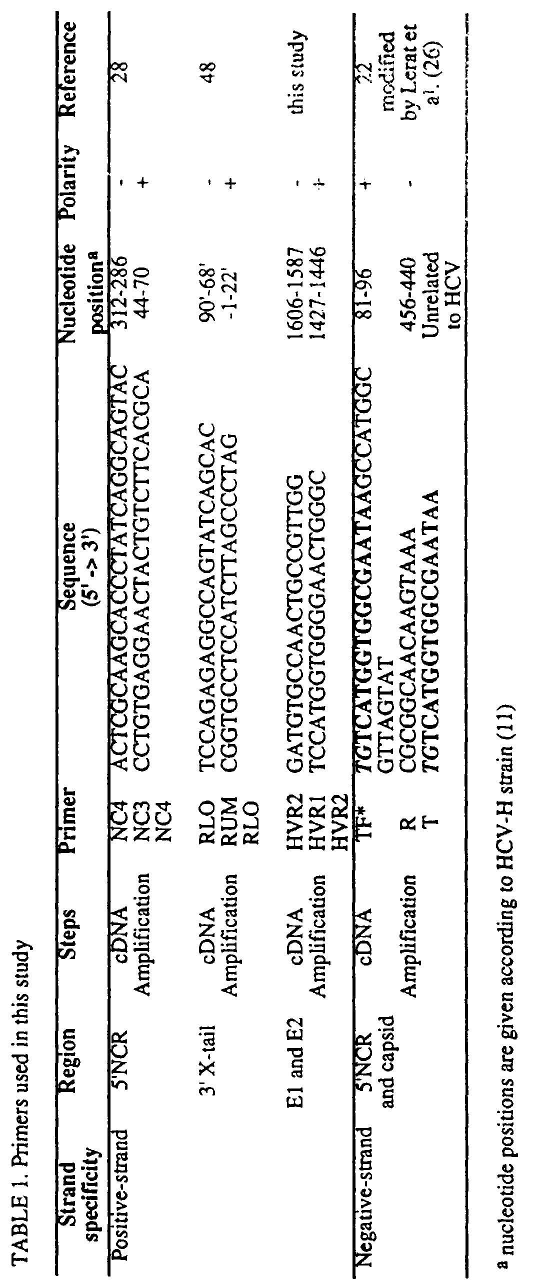

**TABLE 2**

| Characteristics of the sera used for inoculation | | | | | | | | | | |
|---|---|---|---|---|---|---|---|---|---|---|
| | | | | anti-HVC antibodies | | | | *in vitro* infectivity | | |
| serum | genotype | titre (MEq/ml) | MOI^{b} (genomic Eq/cell) | anti-E1 | anti-E2 | Anti-C, E, NS3, NS4, NS5 | NOB titers | Liver Donor | | |
| | | | | | | | | A | B | C |
| K03 | 1a | > 1200 | >30 | - | - | + | 0 | + | + | + |
| S05 | 1b | 17.8 | 0.44 | + | + | + | 1/2000 | + | + | - |
| L02 | 2a/2c | 39.1 | 0.98 | +++ | +++ | NA^{c} | 1/2500 | - | - | - |

| | | | | | | | | | | |
|---|---|---|---|---|---|---|---|---|---|---|
| ^{b}MOI, a viremia superior to 40 MEq/ml (bDNA quantification) gives a Multiplicity Of Infection (MOI) of 1 genomic equivalent per cell under our conditions of infection | | | | | | | | | | |
| ^{c}NA, not available | | | | | | | | | | |
| ^{d}ND, not determined | | | | | | | | | | |

### REFERENCES

1. Choo, Q. L., G. Kuo, A. J. Weiner, L. R. Overby, D. W. Bradley, and M. Houghton. 1989. Isolation of a cDNA clone derived from a blood-borne non-A, non-B viral hepatitis genome. Science 244:359-362.
3. Cribier, B., C. Schmitt, A. Bingen, A. Kim, and F. Keller. 1995. in vitro infection of peripheral blood mononuclear cells by hepatitis C virus. J. Gen. Virol. 76:2485-2491.
4. Friedman-Alvermann, B., D. Muller, and K. Spies. 1985. Isolation and propagation of hepatitis A virus in hepatoma cell cultures. Acta Virol. 29:482-486.
5. Gregersen, J. P., S. Mehdi, and R. Mauler. 1988. Adaptation of hepatitis A virus to high titre growth in diploid and permanent cell cultures. Med. Microbiol. Immunol. (Berl.) 177:91-100.
6. Gretch, D. R., S. J. Polyak, J. J. Wilson, R. L. Carithers, J. D. Perkins, and L. Corey. 1996. Tracking hepatitis C virus quasispecies major and minor variants in symptomatic and asymptomatic liver transplant recipients. J. Virol. 70:7622-7631.
7. Gripon, P., C. Diot, N. Thézé, I. Fourel, O. Loréal, C. Bréchot, and C. Guguen-Guillouzo. 1988. Hepatitis B virus infection of adult human hepatocytes cultured in the presence of dimethylsulfoxide. J. Virol. 34:449-460.
8. Guillouzo, A., and C. Guguen-Guillouzo. 1986. Isolated and cultured hepatocytes. John Libbey Eurotext Ltd (ed.), Montrouge.
9. Guguen-Guillouzo, C. 1992. Isolation and culture of animal and human hepatocytes, p. 197-223. In R. I. Freshney (ed.), Culture of epithelial cells. Wiley-Liss, Inc., New York.
10. Iacovacci, S., A. Manzin, S. Barca, M. Sargiacomo, A. Serafino, M. B. Valli, G. Macioce, H. J. Hassan, A. Ponzetto, M. Clementi, C. Peschle, and G. Carloni. 1997. Molecular characterization and dynamics of hepatitis C virus replication in human fetal hepatocytes infected in vitro. Hepatology 26:1328-1337.
11. Inchauspé, G., S. Zebedee, D. H. Lee, M. Sugitani, M. Nasoff, and A. M. Prince. 1991. Genomic structure of the human prototype strain H of the hepatitis C virus: Comparison with American and Japanese isolates. Proc. Natl. Acad. Sci. USA 88: 10292-10296.
12. Isom, H. C., T. Secott, I. Georgoff, C. Woodworth, and J. Mummaw. 1985. Maintenance of differentiated rat hepatocytes in primary culture. Proc. Natl. Acad. Sci. USA 82:3252-3256.
13. Ito, T., J. Mukaigawa, J. Zuo, Y. Hirabayashi, K. Mitamura, and K. Yasui. 1996. Cultivation of hepatitis C virus in primary hepatocyte culture from patients with chronic hepatitis C results in release of high titer infectious virus. J. Gen. Virol. 77:1043-1054.
14. Kaito, M., S. Watanabe, K. Tsukiyama-Kohara, K. Yamaguchi, Y. Kobayashi, M. Konishi, M. Yokoi, S. Ishida, S. Suzuki, and M. Kohara. 1994. Hepatitis C virus particle detected by electron microscopic study. J. Gen. Virol. 75:1755-1760.
15. Kao, J. H., P. J. Chen, M. Y. Lai, T. H. Wang, and D. S. Chen. 1997. Positive and negative strand of hepatitis C virus RNA sequences in peripheral blood mononuclear cells in patients with chronic hepatitis C: No correlation with viral genotypes 1b, 2a, and 2b. J. Med. Virol. 52:270-274.
16. Kato, N., T. Nakazawa, T. Mizutani, and K. Shimotohno. 1995. Susceptibility of human T-lymphotropic virus type I infected cell line MT-2 to hepatitis C virus infection. Biochem. Biophys. Res. Comm. 206:863-869.
17. Kato, N., M. Ikeda, T. Mizutani, K. Sugiyama, M. Noguchi, S. Hirohashi, and K. Shimotohno. 1996. Replication of hepatitis C virus in cultured non-neoplastic human hepatocytes. Jpn. J. Cancer Res. 87:787-792.
18. Kojima, H., T. Shibayama, A. Sato, S. Suzuki, F. Ichida, and C Hamada. 1981. Propagation of human hepatitis A virus in conventional cell lines. J. Med. Virol. 7:273-286.
19. Kolykhalov, A. A., S. M. Feinstone, and C. M. Rice. 1996. Identification of a highly conserved sequence element at the 3' terminus of hepatitis C virus genome RNA. J. Virol. 70:3363-3371.
20. Kwok, S., and R. Higushi. 1989. Avoiding false positives with PCR. Nature (London) 339:237-238.
21. Landford, R. E. , K. D. Carey, L. E. Estlack, G. Con Smith, and R. V. Hay. 1989. Analysis of plasma protein and lipoprotein synthesis in long-term primary cultures of baboon hepatocytes maintained in serum-free medium. In vitro Cell. and Develop. Biol. 25:174-182.
22. Landford, R. B., C. Sureau, J. R. Jacob, R. White, and T. R. Fuerst. 1994. Demonstration of in vitro infection of chimpanzee hepatocytes with hepatitis C virus using strand-specific RT/PCR. Virology 202:606-614.
23. Landford, R. B., D. Chavez, F. V. Chisari, and C. Sureau. 1995. Lack of detection of negative-strand hepatitis C virus RNA in peripheral blood mononuclear cells and other extrahepatic tissues by the highly strand-specific rTth reverse transcriptase PCR. J. Virol. 69:8079-8083.
24. Laskus, T., L. F. Wang, J. Rakela, H. Vargas, A. D. Pinna, A. C. Tsamandas, A. J. Demetris, and J. Fung. 1996. Dynamic behavior of hepatitis C virus in chronically infected patients receiving liver graft from infected donors. Virology 20:171-176.
25. Laskus, T., M. Radkowski, L. F. Wang, J. Cianciara, H. Vargas, and J. Rakela. 1997. Hepatitis C virus negative strand RNA is not detected in peripheral blood mononuclear cells and viral sequences are identical to those in serum: a case against extrahepatic replication. J. Gen. Virol. 78:2747-2750.
26. Lerat, H., F. Berby, M. A. Trabaud, O. Vidalin, M. Major, C. Trépo, and G. Inchauspé. 1996. Specific detection of hepatitis C virus minus strand RNA in hematopoietic cells. J. Clin. Invest. 97:845-851.
27. Lerat, H., S. Rumin, F. Habersetzer, F. Berby, M. A. Trabaud, C. Trépo, and G. Inchauspé. In-vivo tropism of hepatitis C virus genomic sequences in hematopoitic cells: influence of viral load, viral genotype and cell phenotype. Blood, in press.
28. Li, J. S., S. P. Tong, L. Vitvitsky, and C. Trépo. 1995. Single-step nested polymerase chain reaction for detection of different genotypes of hepatitis C virus. J. Med. Virol. 45:151-155.
29. Major, M. E., and S. M. Feinstone. 1997. The molecular virology of hepatitis C. Hepatology 25:1527-1538.
30. Martell, M., J. I. Esteban, J. Quer, V. Vargas, R. Esteban, J. Guardia, and J. Gòmez. 1994. Dynamic behavior of hepatitis C virus quasispecies in patients undergoing orthotopic liver transplantation. J. Virol. 68:3425-3436.
31. Mihm, S., H. Hartmann, and G. Ramadori. 1996. A reevaluation of the association of hepatitis C virus replicative intermediates with peripheral blood cells including granulocytes by a tagged reverse transcription/polymerase chain reaction technique. J. Hepatol. 24:491-497.
32. Miller, R. H., and R. H. Purcell. 1990. Hepatitis C virus shares amino acid sequence similarity with pestiviruses and flaviviruses as well as two plant virus supergroups. Proc. Natl. Acad. Sci. USA 87:2057-2061.
33. Mizutani, T., N. Kato, M. Ikeda, K. Sugiyama, and K. Shimotohno. 1996. Long-term human T-cell culture system supporting hepatitis C virus replication. Biochem. Biophys. Res. Commun. 227:82-826.
34. Nakajima, N., M. Hijikata, H. Yoshikura, and Y. K. Shimizu. 1996. Characterization of long-term cultures of hepatitis C virus. J. Virol. 70:3325-3329.
35. Rosa, D., S. Campagnoli, C. Moretto, E. Guenzi, L. Cousens, M. Chin, C. Dong, A. J. Weiner, J. Y. N. Lau, Q. L. Choo, D. Chien, P. Pileri, M. Houghton, and S. Abrignani. 1996. A quantitative test to estimate neutralizing antibodies to the hepatitis C virus: Cytofluorimetric assessment of envelope glycoprotein 2 binding to target cells. Proc. Natl. Acad. Sci. USA 93:1759-1763.
37. Rumin, S., P. Gripon, J. Le Seyec, M. Corral-Debrinsky, and C. Guguen-Guillouzo. 1996. Long-term productive episomal hepatitis B virus replication in primary cultures of adult human hepatocytes infected in vitro. J. Viral Hepat. 3:227-238.
38. Sansonno, D., A. R. Iacobelli, V. Cornacchiulo, G. Lodice, and F. Dammacco. 1996. Detection of hepatitis C virus (HCV) proteins by imunofluorescence and HCV RNA genomic sequences by non-isotopic in situ hybridization in bone marrow and peripheral blood mononuclear cells of chronically HCV-infected patients. Clin. Exp. Immunol. 103:414-421.
39. Seipp, S., H. M. Mueller, E. Pfaff, W. Stremmel, L. Theilmann, and T. Goeser. 1997. Establishment of persistent hepatitis C virus infection and replication in vitro. J. Gen. Virol. 78:2467-2476.
40. Shimizu, Y. K., A. Iwamoto, M. Hijikata, R. H. Purcell, and H. Yoshikura. 1992. Evidence for in vitro replication of hepatitis C virus genome in a human T-cell line. Proc. Natl. Acad. Sci. USA 89:5477-5481.
41. Shimizu, Y. K., S. M. Feinstone, M. Kohara, R. H. Purcell, and H. Yoshikura. 1996. Hepatitis C virus: detection of intracellular virus particles by electron microscopy. Hepatology 23:205-209.
42. Shimizu, Y. K., H. Igarashi, T. Kanematu, K. Fujiwara, D. C. Wong, R. H. Purcell, and H. Yoshikura. 1997. Sequence analysis of the hepatitis C virus genome recovered from serum, liver, and peripheral blood mononuclear cells of infected chimpanzees. J. Virol. 71:5769-5773.
43. Siegl, C., J. deChastonay, and C Kronauer. 1984. Propagation and assay of hepatitis A virus in vitro. J. Virol. Methods 9:53-67.
44. Smith, D. B., J. McAllister, C. Casino, and P. Simmonds. 1997. Virus 'quasispecies': making a mountain out of a molehill? J. Gen. Virol. 78:1511-1519.
45. Tanaka, T., J. Y. N. Lau, M. Mizokami, E. Orito, E. Tanaka, K. Kiyosawa, K. Yasui , Y. Ohta, A. Hasegawa, S. Tanaka, and M. Kohara. 1995. Simple fluorescent enzyme immunoassay for detection and quantification of hepatitis C viremia. J. Hepatol. 23:742-745.
46. Tanaka, E., K. Kiyosawa, A. Matsumoto, T. Kashiwakuma, A. Hasegawa, H. Mori, O. Yanagihara, and Y. Ohta. 1996. Serum levels of hepatitis C virus core protein in patients with chronic hepatitis C treated with interferon alfa. Hepatology 23:13330-1333.
47. Tanaka, T., N. Kato, M. J. Cho, and K. Shimotohno. 1995. A novel sequence found at the 3' terminus of hepatitis C virus genome. Biochem. Biophys. Res. Comm. 215:744-749.
48. Umlauft, F., D. T. Wong, P. J. Oefner, P. A. Underhill, R. C. Cheung, T. L. Wright, A. A. Kolykhalov, K. Gruenewald, and H. B. Greenberg. 1996. Hepatitis C virus detection by single-round PCR specific for the terminal 3' noncoding region. J. Clin. Microbiol. 34:2552-2558.
49. Yanagi, M., J. Bukh, S. U. Emerson, and R. H. Purcell. 1996. Contamination of commercially available fetal bovine sera with Bovine Viral Diarrhea Virus genomes: Implications for the study of hepatitis C virus in cell cultures. J. Infect. Dis. 174:1324-1327.
50. Yun, Z., L. Barkholt, and A. Sonnerborg. 1997. Dynamic analysis of hepatitis C virus polymorphism in patients with orthotopic liver transplantation. Transplantation 64:170-172.

## Claims

1. Use of a culture medium containing
- one or more mammalian plasma or sera,
- a chemical or biological compound having an antioxydative property and/or differentiating property, such as DMSO, retinoic acid, vitamin, for example vitamin E, or selenium,
- and/or one or more corticoids
for the *in vitro* hepatitis C virus replication in primary mammalian hepatocytes.

2. Process for the *in vitro* hepatitis C virus replication in primary mammalian hepatocytes comprising the step of incubation of a primary mammalian hepatocyte culture medium containing
- one or more mammalian plasma or sera,
- a chemical or biological compound having an antioxydative property and/or differentiating property, such as DMSO, retinoic acid, vitamin, for example vitamin E, or selenium,
- and/or one or more corticoids

3. Process according to claim 2, characterized in that the culture medium contains:
- one or more mammalian plasma or sera,
- a chemical or biological compound having an antioxydative property and/or differentiating property, such as DMSO, retinoic acid, vitamin, for example vitamin E, or selenium.

4. Process according to claim 2 or 3, characterized in that the culture medium is a primary human hepatocyte culture medium containing a non-infected human plasma or serum (i.e. a human serum not infected by HCV), DMSO, and a corticoid chosen from among hydrocortisone derivatives such as hydrocortisone hemisuccinate, or dexamethasone.

5. Process according to claim 4, characterized in that :
- the ratio of non-infected human plasma or serum is from about 1 % to about 10 % by volume of said culture medium, in particular about 1 %, to about 5 %, and preferably about 2 %,
- the ratio of DMSO in said culture medium is from about 0.5 % to about to about 3 % by volume , in particular about 2 %,
- the concentration of the corticoid is from about 10⁻⁷M to about 10⁻⁴M, in particular about 10⁻⁶M.

6. Process according to claim 4 or 5, characterized in that the culture medium also contains :
- antibiotics such as penicillin, streptomycin, and/or
- interferon, and/or
- mammalian insulin, and/or
- foetal calf serum, and/or
- a basic medium for *in vitro* cell cultures, such as William's E medium, or a culture medium adapted for long term culture of epithelial cells.

7. Process according to anyone of claims 2 to 6, characterized in that the culture medium does not contain PEG.

8. Process according to anyone of claims 2 to 7, characterized in that :
- the HCV titer of the inoculum is from about 10⁻³ to about 100 genomic equivalents per hepatocyte of the culture, in particular from 0.1 to about 10 and in particular from about 1 to about 10 genomic equivalents/hepatocyte,
- and the resulting HCV titer in the culture medium after incubation with said inoculum, is from about 5.10⁻⁵ to about 1 genomic equivalent/hepatocyte/day.

9. Process according to anyone of claims 2 to 8, wherein the hepatocytes are initially seeded in the culture medium at the ratio of about 1.5 x10⁵ to about 2.10⁵ cells/cm².

10. Use of a process according to anyone of claims 2 to 9, for the *in vitro* screening of anti-HCV drugs.

11. Use of a process according to anyone of claims 2 to 9, for the i*n vitro* diagnosis of HCV.

12. Use of a process according to anyone of claims 2 to 9, for the preparation of vaccines against HCV.

13. HCV infected mammalian hepatocyte culture medium containing
- one or more mammalian plasma or sera,
- a chemical or biological compound having an antioxydative property and/or differentiating property, such as DMSO, retinoic acid, vitamin, for example vitamin E, or selenium,
- and/or one or more corticoids.

14. HCV infected mammalian hepatocyte culture medium containing
- one or more mammalian plasma or sera,
- a chemical or biological compound having an antioxydative property and/or differentiating property, such as DMSO, retinoic acid, vitamin, for example vitamin E, or selenium.

15. HCV infected mammalian hepatocyte culture medium according to claim 14, characterized in that said culture medium is a human hepatocyte culture medium containing a non-infected human serum or plasma, DMSO, and a corticoid chosen among hydrocortisone derivatives such as hydrocortisone hemisuccinate, or dexamethasone.

16. HCV infected mammalian hepatocyte culture medium according to claim 13 to 15, characterized in that :
- the ratio of non-infected human plasma or serum is from about 1 % to about 10 % by volume of said culture medium, in particular about 1 %, to about 5%, and preferably about 2%,
- the ratio of DMSO in said culture medium is from about 0.5 % to about to about 3 % by volume , in particular about 2 %,
- the concentration of the corticoid is from about 10⁻⁷M to about 10⁻⁴M, in particular about 10⁻⁶M.

17. HCV infected mammalian hepatocyte culture medium according to anyone of claims 13 to 16, wherein HCV infected hepatocytes survive for at least 4 months.

18. HCV particles obtained according to the process of anyone of claims 1 to 9.


SEQUENCE LISTING

(1) GENERAL INFORMATION:

(i) APPLICANT:
(A) NAME: I.N.S.E.R.M.(Institut National de la Recherche
Médicale)
(B) STREET: 101, rue de Tolbiac
(C) CITY: Paris
(E) COUNTRY: FRANCE
(F) POSTAL CODE (ZIP): 75654 CEDEX 13

(ii) TITLE OF INVENTION: PROCESS FOR THE IN VITRO REPLICATION OF HCV

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98401554.5

(2) INFORMATION FOR SEQ ID NO: 1:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 1:

ACTCGCAAGC ACCCTATCAG GCAGTAC 27

(2) INFORMATION FOR SEQ ID NO: 2:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 2:

CCTGTGAGGA ACTACTGTCT TCACGCA 27

(2) INFORMATION FOR SEQ ID NO: 3:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 3:

TCCAGAGAGG CCAGTATCAG CAC 23

(2) INFORMATION FOR SEQ ID NO: 4:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 4:

CGGTGCCTCC ATCTTAGCCC TAG 23

(2) INFORMATION FOR SEQ ID NO: 5:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 5:

GATGTGCCAA CTGCCGTTGG 20

(2) INFORMATION FOR SEQ ID NO: 6:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 6:

TCCATGGTGG GGAACTGGGC 20

(2) INFORMATION FOR SEQ ID NO: 7:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 7:

TGTCATGGTG GCGAATAAGC CATGGCGTTA GTAT 34

(2) INFORMATION FOR SEQ ID NO: 8:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 8:

CGCGGCAACA AGTAAA 16

(2) INFORMATION FOR SEQ ID NO: 9:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 9:

TGTCATGGTG GCGAATAA 18